(19)

Europäisches
Patentamt
European
Patent Office
Office européen
des brevets

(11) **EP 2 653 853 A1**

(12) **EUROPEAN PATENT APPLICATION**
published in accordance with Art. 153(4) EPC

(43) Date of publication:
**23.10.2013 Bulletin 2013/43**

(21) Application number: **11848652.1**

(22) Date of filing: **06.12.2011**

(51) Int Cl.:
**G01N 21/27** *(2006.01)*  **G01N 33/543** *(2006.01)*

(86) International application number:
**PCT/JP2011/078123**

(87) International publication number:
**WO 2012/081445 (21.06.2012 Gazette 2012/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.12.2010 JP 2010279000**

(71) Applicant: **Konica Minolta, Inc.**
**Tokyo 100-7015 (JP)**

(72) Inventors:
• **IZUMIYA, Naoki**
  **Hachioji-shi**
  **Tokyo 192-8505 (JP)**
• **YOSHIHARA, Yuka**
  **Hachioji-shi**
  **Tokyo 192-8505 (JP)**

(74) Representative: **Gille Hrabal**
**Patentanwälte**
**Brucknerstrasse 20**
**40593 Düsseldorf (DE)**

(54) **INTERMOLECULAR INTERACTION MEASUREMENT METHOD, MEASUREMENT SYSTEM FOR USE IN THE METHOD, AND PROGRAM**

(57)  The purpose of the present invention is to efficiently obtain a correct value that indexes the degree of progression of an intermolecular interaction between biological molecules or organic polymers. Provided are: a method for optically measuring an intermolecular interaction among multiple substances on a thin film, which comprises detecting the intensity of light reflected on the thin film (reflected light) or light transmitted through the thin film (transmitted light) at a first wavelength before the occurrence of the intermolecular interaction by a detection means for detecting the reflected light or the transmitted light, detecting the intensity of the reflected light or the transmitted light at the first wavelength after the occurrence of the molecular interaction by the detection means, and calculating the change in intensity of the reflected light or the transmitted light before and after the occurrence of the intermolecular interaction at the first wavelength; a measurement system for use in the method; and a program by which a processing for the measurement can be performed on a computer.

*FIG.6*

## Description

TECHNICAL FIELD

The present invention relates to a measuring method, a measuring system and a measuring program of intermolecular interactions, in particular, to a measuring method of intermolecular interactions being capable of generating a value as an index indicating a degree of progress in the intermolecular interactions of biomolecules and organic polymers etc., a measurement system for the method, and a program that causes a computer to execute a process for such measurement.

BACKGROUND ART

Labeling by radioactive or fluorescent substances has generally been employed for conventional measurements of bonds or links such as intermolecular interactions between biomolecules, e.g., antigen-antibody reactions and intermolecular interactions between organic polymers. This labeling, however, is time-consuming, and especially labeling of proteins requires complicated procedures and causes denaturation of the proteins in some cases. To address this, recently, RIfS (Reflectometric interference spectroscopy) making use of a change in interference color of an optical thin film has been proposed and already put into practical use as simplified means for directly detecting bonds or links between biomolecules and organic polymers without labeling. The principle of the RIfS is disclosed in Patent Document 1 and Non-Patent Document 1, for example.

RIfS will now be briefly described. RIfS uses a substrate 102 provided with an optical thin film 104 as illustrated in FIGS. 18A, 18B and 18C. When the optical thin film 104 on the substrate 102 is irradiated with white light as illustrated in FIG. 18A, the spectral intensities of the white light itself and the reflected light thereof are depicted with solid lines 106 and 108, respectively, as shown in a typical example of FIG. 19. A reflection spectrum 110 represented by a solid line and having the bottom (the lowest point of the spectrum curve) can be derived by obtaining reflectance from the spectral intensities of the incident white light and the reflected light, as illustrated in FIG. 20.

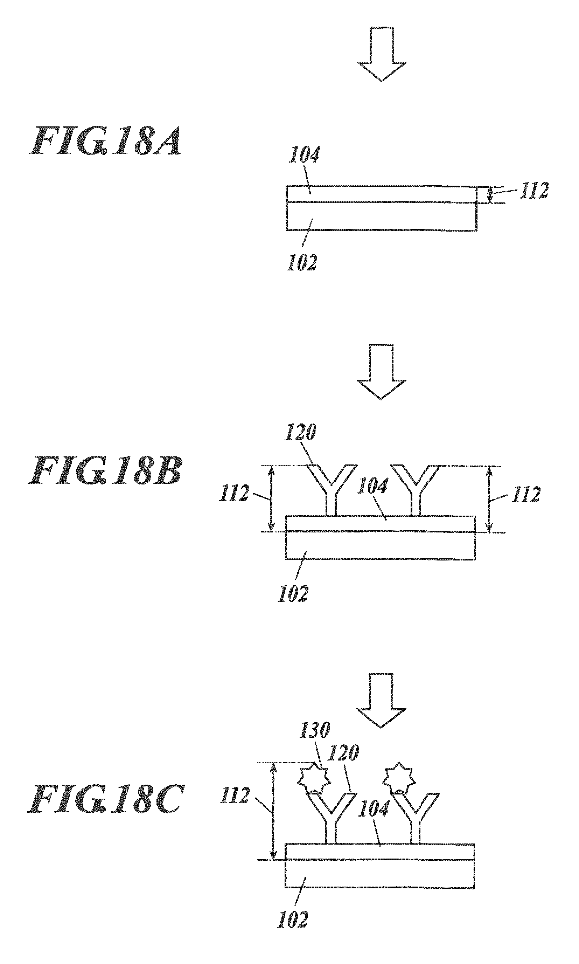

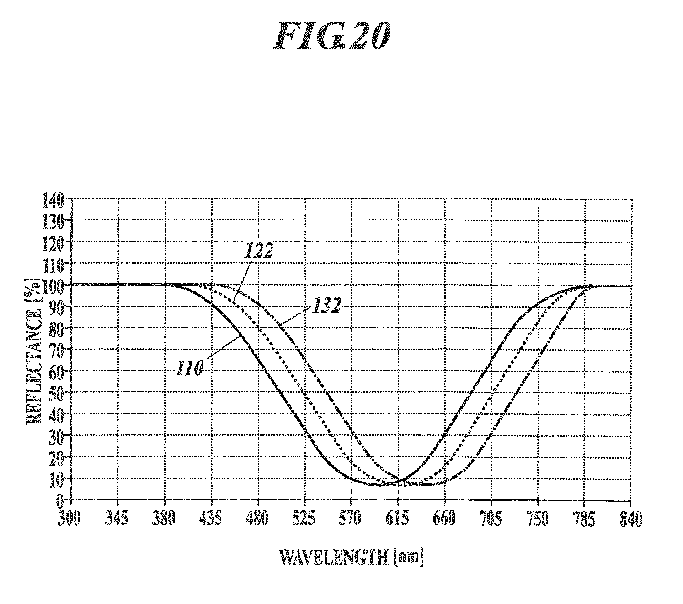

Ligand molecules 120 are provided on the optical thin film 104 in order to detect intermolecular interactions, as illustrated in FIG. 18B. The ligand molecules 120 provided on the optical thin film 104 change the optical thickness 112 of the part on which the ligand molecules 120 are provided, leading to a change in optical path length, and the interference wavelength is also changed due to reflection interference. This causes a peak shift of the spectral intensity distribution of the reflected light, and as a result, the reflection spectrum 110 shifts to a reflection spectrum 122 (dotted line), as illustrated in FIG. 20. A sample solution is then poured over the optical thin film 104, so that binding occurs between the ligand molecules 120 and analyte molecules 130 in the sample solution, as illustrated in FIG. 18C. The binding of the ligand molecules 120 and the analyte molecules 130 further modifies the optical thickness 112 of the part to which the analyte molecules 130 are bound. Partial attachment of the analyte molecules 130 onto the ligand molecules 120 generates a non-uniform layer; however, the non-uniform layer can be macroscopically regarded as a uniform layer having a predetermined optical thickness dependent on the number of attached analyte molecules 130. Thus, the optical thickness of the uniform layer through which incident light passes varies depending on the number of the attached analyte molecules 130. As a result, the reflection spectrum 122 shifts to a reflection spectrum 132 (dashed line), as illustrated in FIG. 20. The detection of variation between the bottom wavelength (wavelength having the minimum reflectance) of the reflection spectrum 132 and the bottom wavelength of the reflection spectrum 122 enables intermolecular interactions to be detected. Furthermore, the detection of a difference between the bottom wavelength of the reflection spectrum 132 and the bottom wavelength of the reflection spectrum 122 enables the degree of progress in intermolecular interactions to be detected.

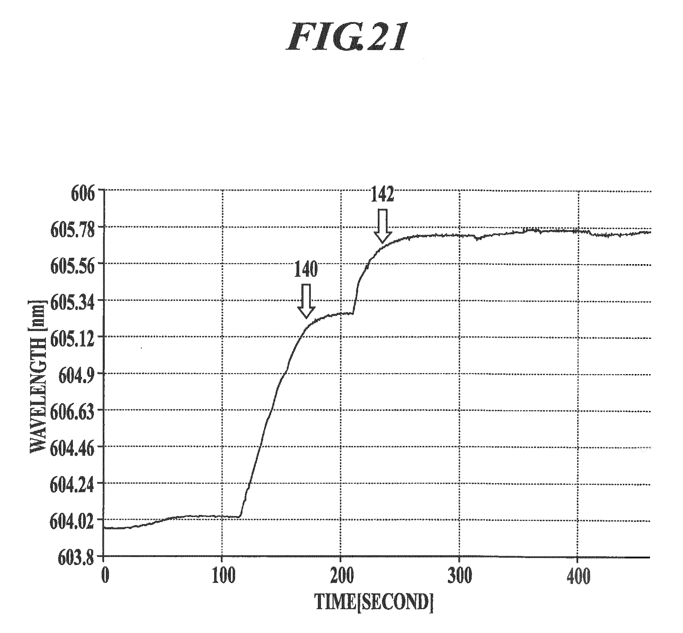

In a temporal transition of the variation in the bottom wavelengths, as shown in FIG. 21, the variation in the bottom wavelength by the ligand 120 can be observed at a time point 140, which is a first shoulder of the curve, while the variation in the bottom wavelength by the bonds or links between the ligand 120 and the analyte 130 can be observed at a time point 142, which is a second shoulder of the curve.

PRIOR ART DOCUMENTS

PATENT DOCUMENTS

[Patent Document 1] Japanese Patent No. 3786073

Non-Patent Documents

[Non- Patent Document 1] Sandstrom et al., APPL. OPT., 24, 472, 1985

DISCLOSURE OF THE INVENTION

PROBLEMS TO BE SOLVED BY THE INVENTION

Unfortunately, the study of the present inventors demonstrates that observing a temporal transition of the variation in bottom wavelengths, in principle, has a limitation of the accuracy in measurement and thus the method is unsatisfactory for more accurate determination of the degree of progress in intermolecular interactions. This is for the following reasons.

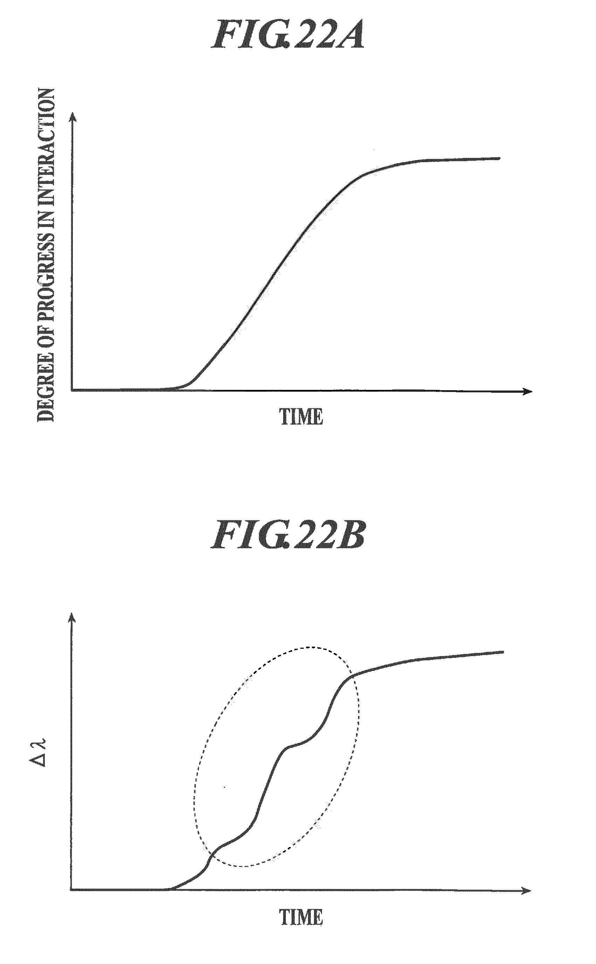

Bonds or links such as intermolecular interactions between biomolecules, e.g., antigen- antibody reactions, and intermolecular interactions between organic polymers generally proceed evenly, albeit microscopic repetitions of binding and leaving. That is, intermolecular interactions proceed over time, as illustrated in FIG. 22A.

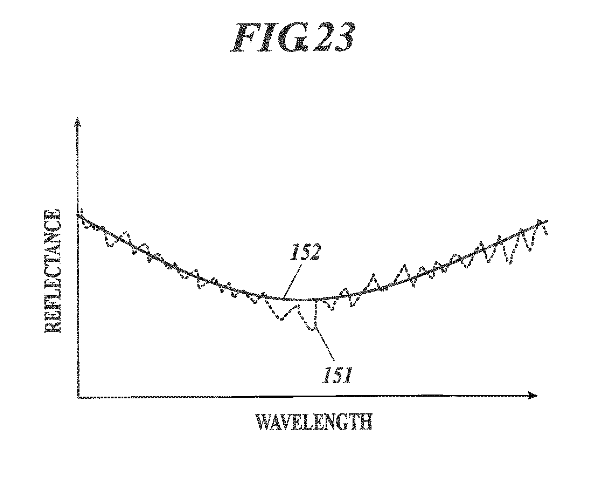

However, actual data output from a detector for determining the spectral intensity of the reflected light has repeated minute fluctuations, for example, like reflectance data 151 shown in FIG. 23, and thus, arithmetic is necessary in order to determine the bottom position; for example, an approximate curve 152 is calculated from the reflectance data 151 by a fitting procedure, and then the minimum value of the approximate curve is determined as the above- described bottom position. In addition, a variation in reflectance near the bottom is small, so that the above method which uses an approximate curve to determine the bottom position tends to determine an inaccurate bottom position in principle, and in particular, such a method is unsuitable for more accurate calculation of the degree of progress in intermolecular interactions. As a result, the difference $\Delta\lambda$ in bottom wavelengths may vary differently from the degree of progress in the intermolecular interactions as illustrated in FIG. 22B, and in such a case, the difference $\Delta\lambda$ in bottom wavelengths is an inappropriate value for the accurate determination of the degree of progress in intermolecular interactions.

Furthermore, the method of tracing a change in the difference $\Delta\lambda$ in bottom wavelengths only traces a shift of a single point on the graph of the spectral characteristics of the reflected light and does not detect a general shift of the graph, which limits accurate detection of variation.

On top of that, the above- described calculation of the approximate curve may need a high- performance arithmetic unit or a complicated arithmetic, or the speed of arithmetic may be insufficient in a high- rate intermolecular interaction.

An object of the present invention, which has been made in view of such a conventional problem, is to provide a measuring method of intermolecular interactions, the method being capable of accurately and efficiently generating a value as an index indicating the degree of progress in intermolecular interactions of biomolecules and organic polymers, a measuring system for the method, and a program for causing a computer to execute a process for such measurement.

MEANS FOR SOLVING THE PROBLEM

The invention described in claim 1 for solving the above problems is a measuring method of an intermolecular interaction for optically measuring an intermolecular interaction between a plurality of substances on a thin film, the method comprising detecting intensity at a first wavelength of reflected light or transmitted light before start of the intermolecular interaction with a detection unit which detects the reflected light from the thin film or the transmitted light transmitted through the thin film; detecting intensity at the first wavelength of the reflected light or the transmitted light after the start of the intermolecular interaction with the detection unit; and calculating a difference in intensity of the reflected light or the transmitted light at the first wavelength between before and after the start of the intermolecular interaction.

The invention described in claim 2 is the measuring method of an intermolecular interaction of claim 1, wherein the intensity of the reflected light is spectral intensity of the reflected light or a value containing the spectral intensity of the reflected light as a variable, or the intensity of the transmitted light is spectral intensity of the transmitted light or a value containing the spectral intensity of the transmitted light as a variable.

The invention described in claim 3 is the measuring method of an intermolecular interaction of claim 1 or 2, further comprising calculating a difference in intensity of the reflected light or the transmitted light at a second wavelength which is different from the first wavelength.

The invention described in claim 4 is the measuring method of an intermolecular interaction of claim 1 or 2, further comprising measuring a plurality of differences in intensity of the reflected light or the transmitted light at different wavelengths within a predetermined wavelength range and calculating a sum of the differences or a sum of absolute values of the differences.

The invention described in claim 5 is a measuring system of an intermolecular interaction for optically measuring an intermolecular interaction between a plurality of substances on a thin film, the system comprising a detection unit which detects reflected light from the thin film or transmitted light transmitted through the thin film, the detection unit detecting intensity at a first wavelength of the reflected light or the transmitted light before start of the intermolecular interaction, and detecting intensity at the first wavelength of the reflected light or the transmitted light after the start of the intermolecular interaction; and an arithmetic unit which calculates a difference in intensity of the reflected light or the transmitted light at the first wavelength between before and after the start of the intermolecular interaction.

The invention described in claim 6 is the measuring system of an intermolecular interaction of claim 5, wherein the intensity of the reflected light is spectral intensity of the reflected light or a value containing the spectral intensity of the reflected light as a variable, or the intensity of the transmitted light is spectral intensity of the transmitted light or a value containing the spectral intensity of the transmitted light as a variable.

The invention described in claim 7 is the measuring system of an intermolecular interaction of claim 5 or 6, wherein the arithmetic unit calculates a difference in intensity of the reflected light or the transmitted light at a second wavelength which is different from the first wavelength.

The invention described in claim 8 is the measuring system of an intermolecular interaction of claim 5 or 6, wherein the arithmetic unit measures a plurality of differences in intensity of the reflected light or the transmitted light at different wavelengths within a predetermined wavelength range to calculate a sum of the differences or a sum of absolute values of the differences.

The invention described in claim 9 is a program for causing a computer to execute a process for optically measuring an intermolecular interaction between substances on a thin film, the program causing the computer to execute a process of detecting intensity at a first wavelength of reflected light or transmitted light before start of the intermolecular interaction with a detection unit for detecting the reflected light from the thin film or the transmitted light transmitted through the thin film; a process of detecting intensity at the first wavelength of the reflected light or the transmitted light after the start of the intermolecular interaction with the detection unit; and a process of calculating a difference in intensity of the reflected light or the transmitted light at the first wavelength between before and after the start of the intermolecular interaction.

The invention described in claim 10 is the program of claim 9, wherein the intensity of the reflected light is spectral intensity of the reflected light or a value containing the spectral intensity of the reflected light as a variable, or the intensity of the transmitted light is spectral intensity of the transmitted light or a value containing the spectral intensity of the transmitted light as a variable.

The invention described in claim 11 is the program of claim 9 or 10, further causing the computer to execute a process of calculating a difference in intensity of the reflected light or the transmitted light at a second wavelength which is different from the first wavelength.

The invention described in claim 12 is the program of claim 9 or 10, further causing the computer to execute a process of measuring a plurality of differences in intensity of the reflected light or the transmitted light at different wavelengths within a predetermined wavelength range to calculate a sum of the differences or a sum of absolute values of the differences.

EFFECTS OF THE INVENTION

According to the present invention, a variation is calculated between the intensity of the reflected light or the transmitted light at a first wavelength before the start of intermolecular interactions and the intensity of the reflected light or the transmitted light at the first wavelength after the start of the intermolecular interactions, so that an exact value as an index indicating the degree of progress in the intermolecular interactions can be efficiently determined by using the difference as an index indicating the degree of progress in the intermolecular interactions.

BRIEF DESCRIPTION OF DRAWINGS

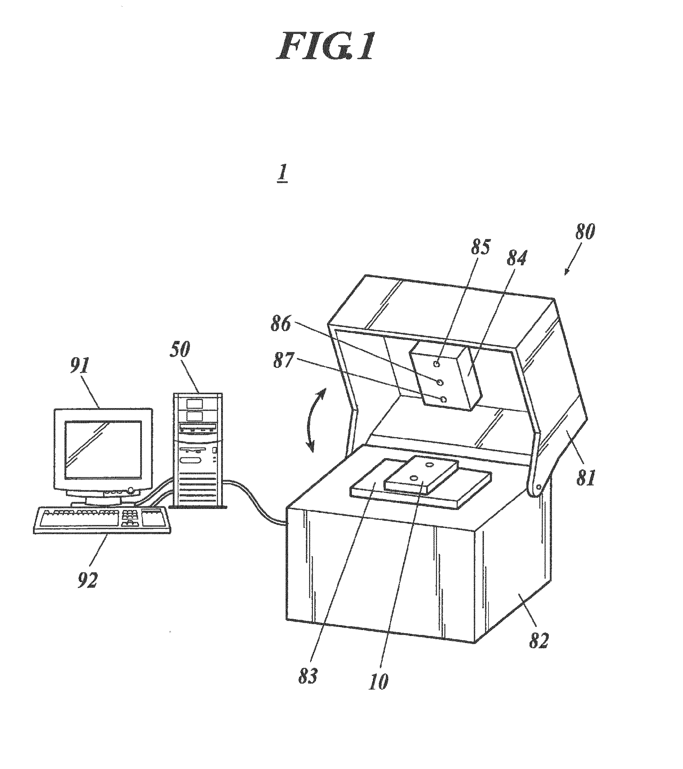

[FIG. 1] This is a schematic diagram illustrating an outline configuration of a measuring system of intermolecular interactions in an embodiment of the present invention.

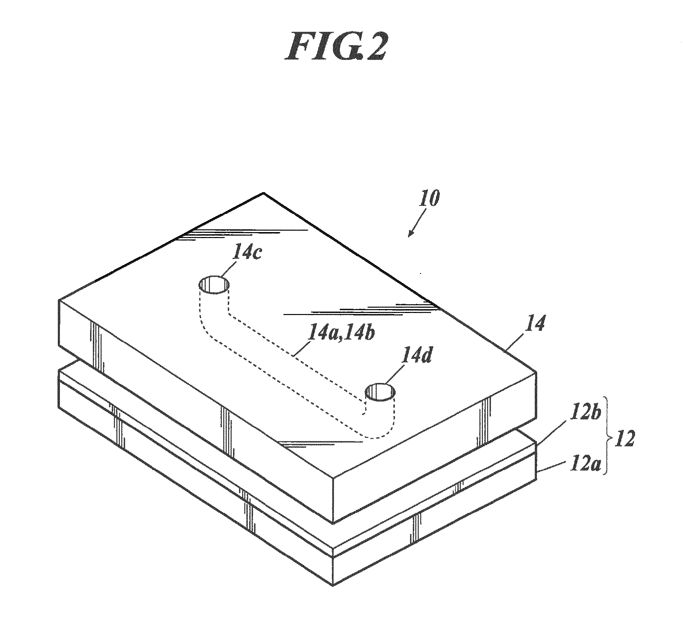

[FIG. 2] This is a perspective view illustrating an outline configuration of a measuring member in an embodiment of the present invention.

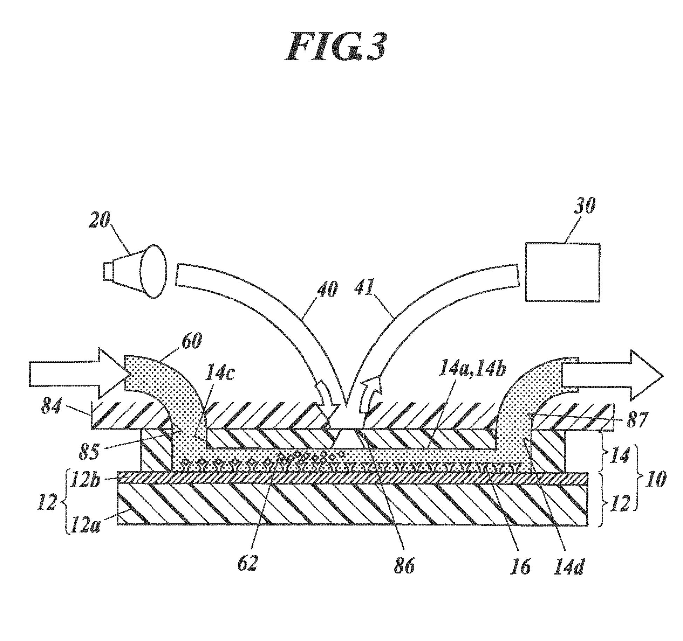

[FIG. 3] This is a schematic diagram illustrating the measurement carried out by the measuring system of intermolecular interactions.

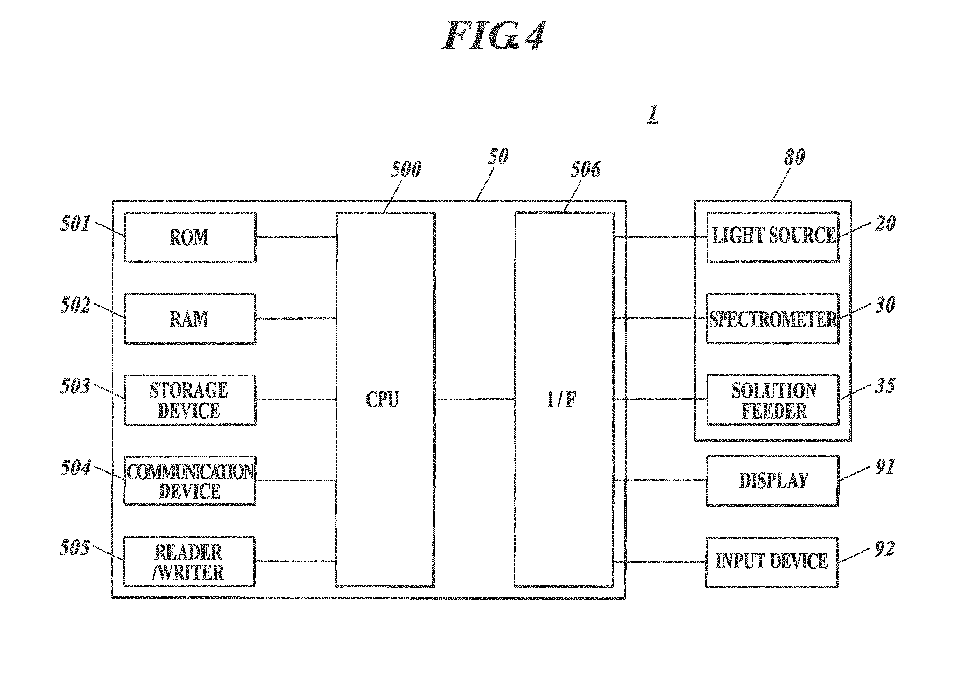

[FIG. 4] This is a circuit block diagram of the measuring system of intermolecular interactions.

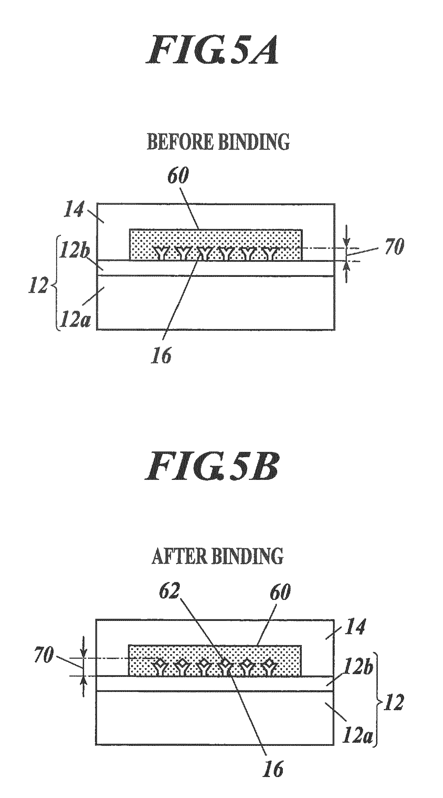

[FIG. 5A] This is a schematic cross-sectional view illustrating binding between a ligand and an analyte (before the binding) in an embodiment of the present invention.

[FIG. 5B] This is a schematic cross-sectional view illustrating binding between a ligand and an analyte (after the binding) in an embodiment of the present invention.

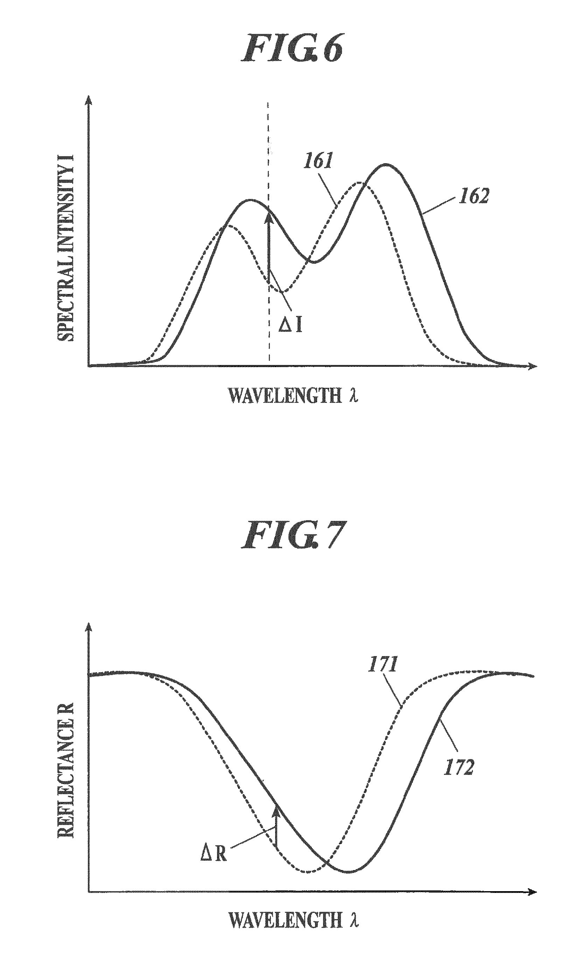

[FIG. 6] This is a graph of an example spectral intensity distribution of reflected light in an embodiment of the present invention, illustrating the calculation of a difference ΔI.

[FIG. 7] This is a graph of an example spectral reflectance distribution of reflected light in an embodiment of the present invention, illustrating the calculation of a difference ΔR.

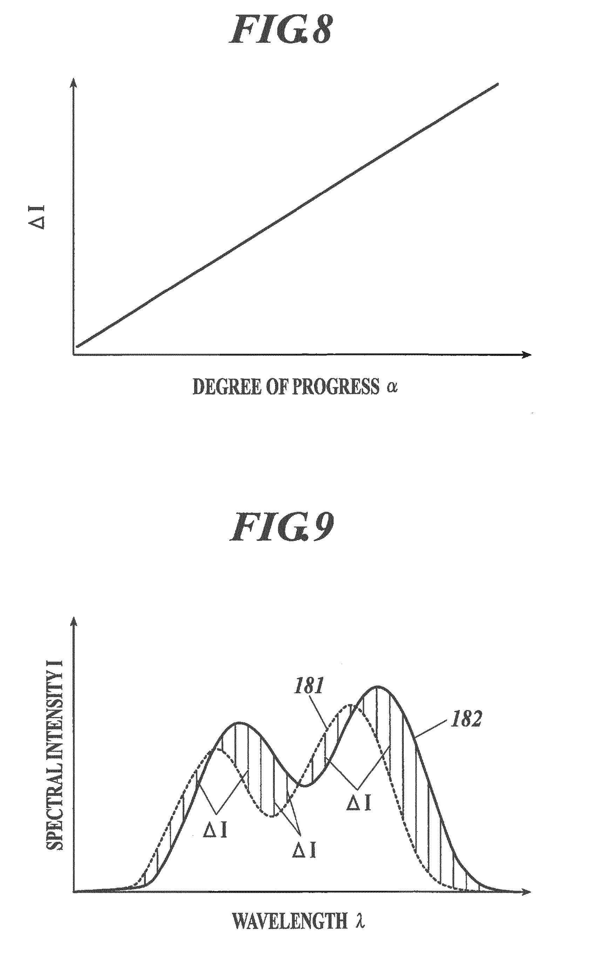

[FIG. 8] This is a graph showing variation of differences ΔI in spectral intensity versus the degree of progress α in intermolecular interactions.

[FIG. 9] This is a graph of an example spectral intensity distribution of reflected light in an embodiment of the present invention, illustrating the calculation of the difference ΔI on two or more different wavelengths.

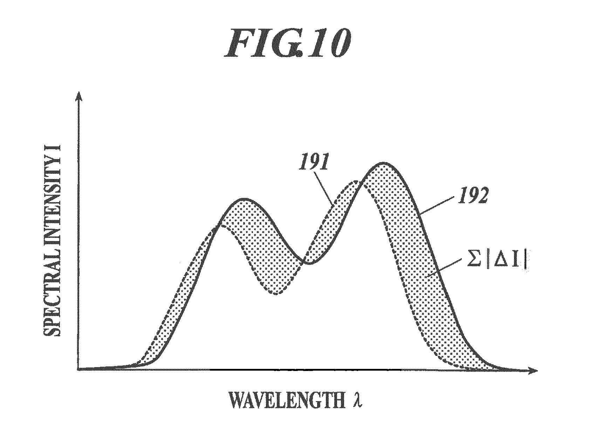

[FIG. 10] This is a graph of an example spectral intensity distribution of reflected light in an embodiment of the present invention, illustrating the calculation of a sum of absolute values of the differences ΔI within a predetermined wavelength range.

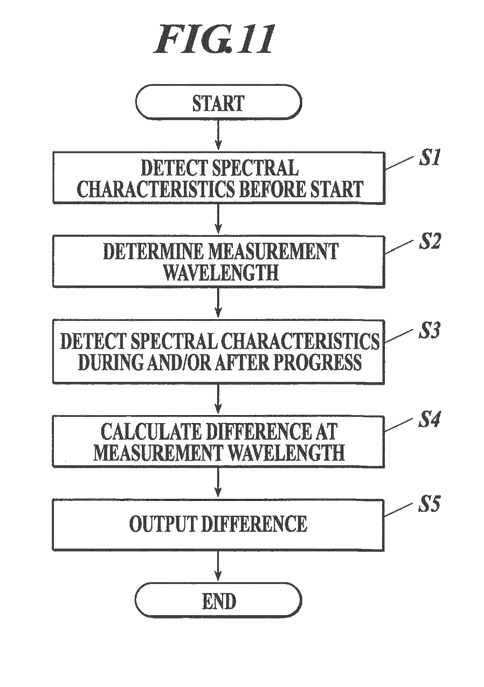

[FIG. 11] This is a flow chart illustrating a process carried out by an arithmetic and control unit in an embodiment of the present invention.

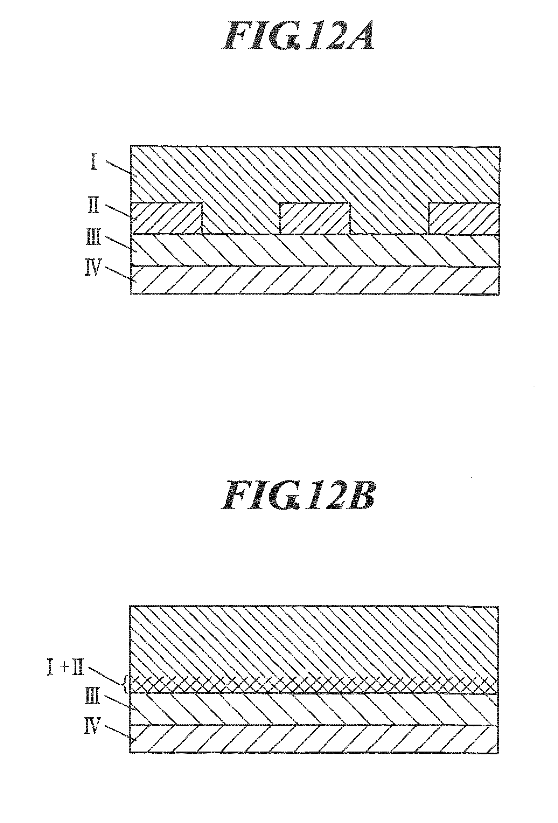

[FIG. 12A] This is a schematic longitudinal cross-sectional view of a space where an intermolecular interaction proceeds, illustrating the measuring principle of the embodiment.

[FIG. 12B] This is a schematic diagram of a virtual space where an intermolecular interaction proceeds, illustrating the measuring principle of the embodiment.

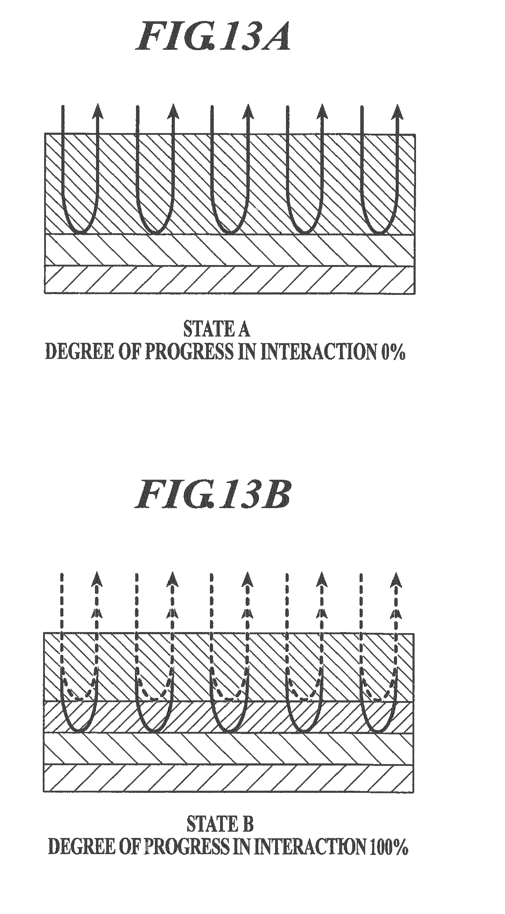

[FIG. 13A] This is a schematic longitudinal cross-sectional view of a space where an intermolecular interaction proceeds, illustrating the measuring principle of the embodiment. The state of 0% degree of progress is schematically illustrated.

[FIG. 13B] This is a schematic longitudinal cross-sectional view of a space where an intermolecular interaction proceeds, illustrating the measuring principle of the embodiment. The state of 100% degree of progress is schematically illustrated.

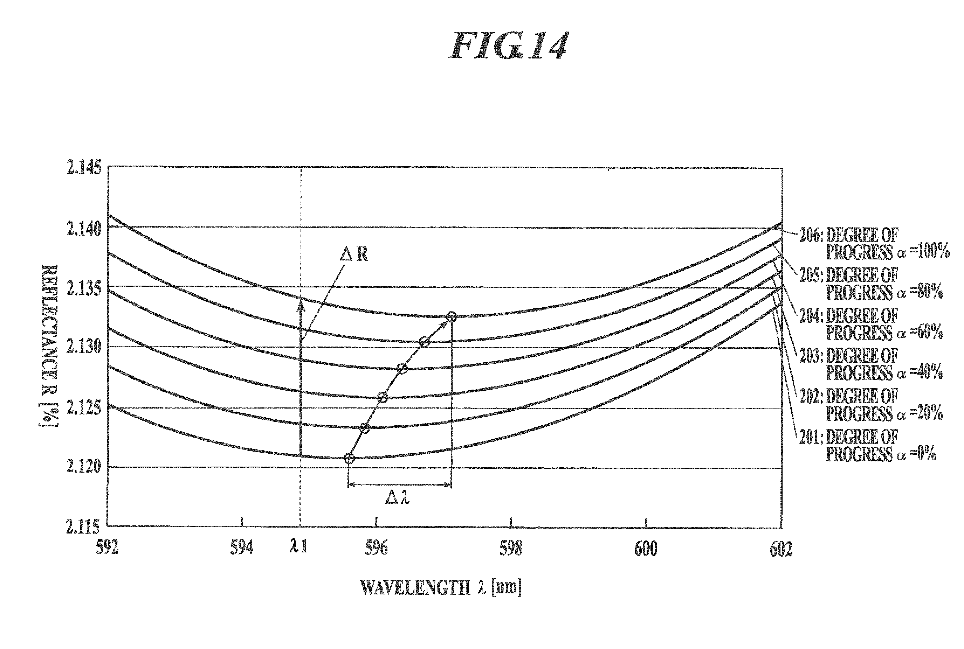

[FIG. 14] This is a graph illustrating spectral reflectance curves at different degrees of progress in intermolecular interactions according to the measuring principle of the embodiment.

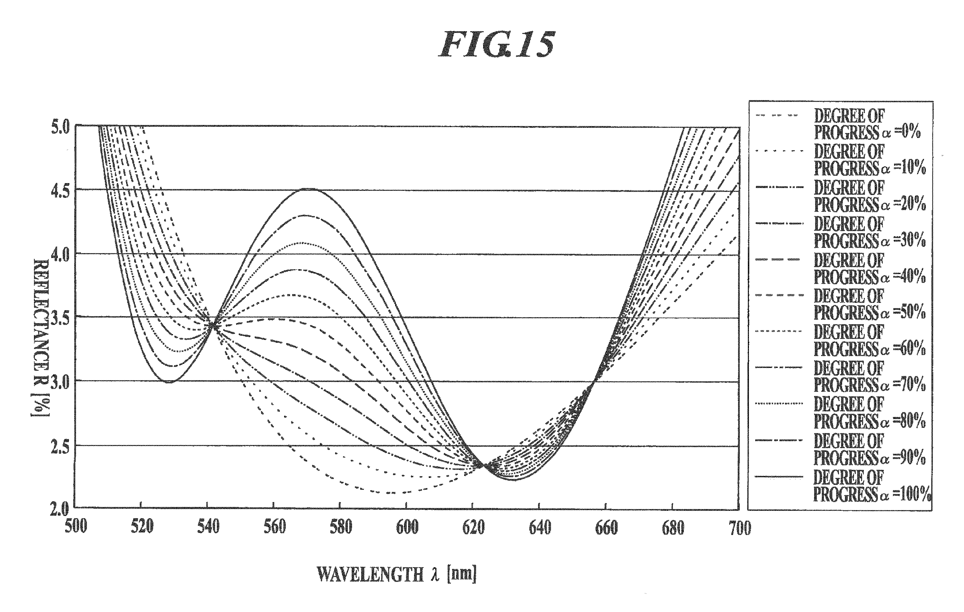

[FIG. 15] This is a graph illustrating spectral reflectance curves at different degrees of progress based on a simulation for supporting the theory of the present invention.

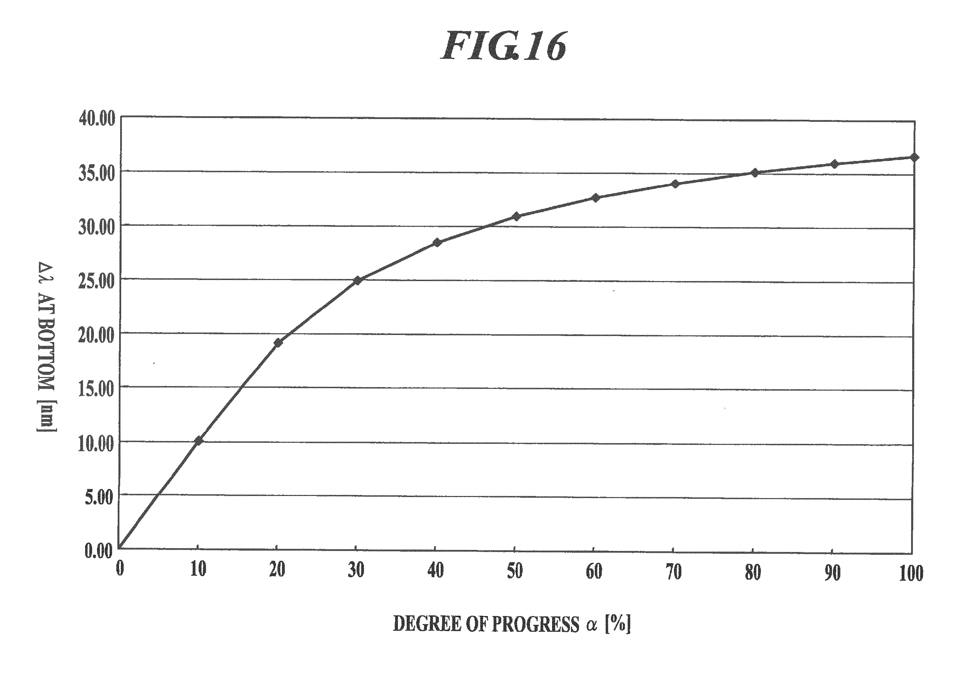

[FIG. 16] This is a graph illustrating the relationship between the degree of progress and the difference Δλ in the bottom wavelength, based on the simulation.

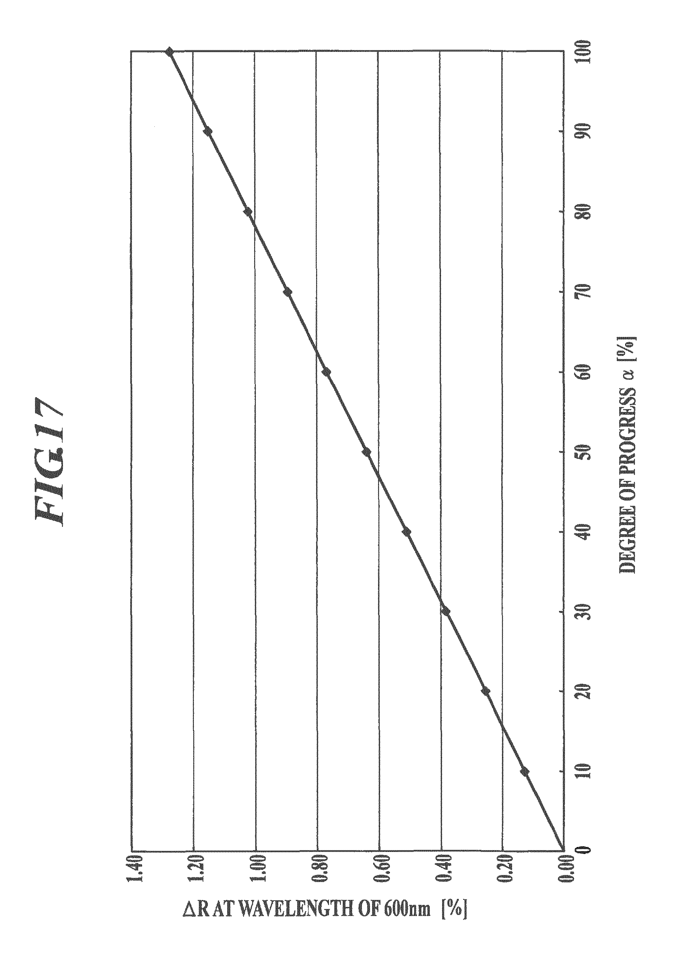

[FIG. 17] This is a graph illustrating the relationship between the degree of progress and the difference ΔR in reflectance, based on the simulation.

[FIG. 18A] This is a schematic diagram illustrating a RIfS scheme.

[FIG. 18B] This is a schematic diagram illustrating the RIfS scheme.

[FIG. 18C] This is a schematic diagram illustrating the RIfS scheme.

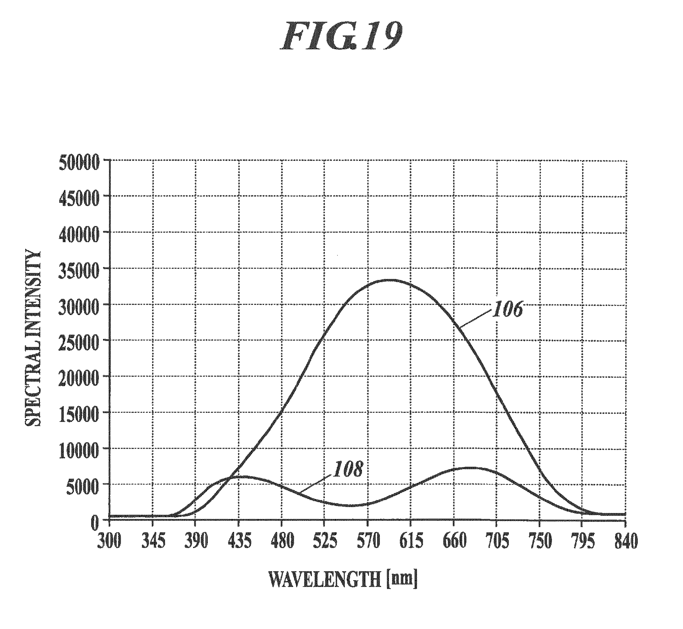

[FIG. 19] This is an example graph illustrating a general relationship between the wavelength and the spectral intensity.

[FIG. 20] This is an example graph illustrating a general relationship between the wavelength and the reflectance.

[FIG. 21] This is an example graph illustrating a schematic variation in the bottom wavelength.

[FIG. 22A] This is a graph illustrating a temporal variation of the degree of progress in intermolecular interactions.

[FIG. 22B] This is an example graph illustrating changes in the difference Δλ over time in the bottom wavelength.

[FIG. 23] This is an example graph illustrating detected data of spectral reflectance and its approximate curve.

EMBODIMENTS FOR CARRYING OUT THE INVENTION

An embodiment of the present invention will now be described with reference to the accompanying drawings; however, the embodiment shall not limit the scope of the present invention.

With reference to FIG. 1, a measuring system 1 of intermolecular interactions is configured by including a measuring unit 80 having a measuring member 10 for holding a sample to be measured and measuring mechanisms such as a light source and a spectrometer described later, an arithmetic and control unit 50 which is a computer connected to the measuring unit 80, and a display 91 and an input device 92 which are connected to the arithmetic and control unit 50. The arithmetic and control unit 50 in the measuring system 1 serves as a control unit of the measuring mechanisms such as the light source and the spectrometer incorporated in the measuring unit 80, an arithmetic unit for detected information, and an input/output unit (interface) for outputting and inputting a control instruction and the detected information.

The measuring unit 80 includes a lower housing 82 and an upper housing 81 pivotally supported by the lower housing 82. The lower housing 82 is provided with a table 83 for holding the measuring member 10. A connection portion 84 is provided on the inner surface of the upper housing 81. The connection portion 84 has an injection orifice 85 and a suction orifice 87, which are to be connected to the measuring member 10 for providing the flow of a sample, and a detection window 86. The upper housing 81 includes a white-light source 20, a spectrometer 30, and optical fibers 40 and 41 as described later. In the upper housing 81, light is emitted or received through the detection window 86. In the preparation for measurement, the upper housing 81 is pivotally moved upwardly to open the table 83 of the lower housing 82 and then the measuring member 10 is placed on the table 83. The upper housing 81 is then moved downwardly to close the unit, and thereby the injection orifice 85 and the suction orifice 87 are connected to the measuring member 10 and the detection window 86 faces the measuring member 10. The preparation for measurement is thereby completed.

As illustrated in FIG. 2, the measuring member 10 includes a sensor chip 12 having an optical thin film, and a flow cell 14 which defines a fluid passage together with the sensor chip 12. The sensor chip 12 includes a silicon substrate 12a. A SiN film 12b (silicon nitride) is evaporated on the silicon substrate 12a. The SiN film 12b is an exemplary optical thin film. The flow cell 14 is a transparent member made of silicone rubber. The flow cell 14 has a channel 14a. Close contact of the flow cell 14 to the sensor chip 12 defines a closed fluid passage 14b, as illustrated in FIG. 3. Both ends of the channel 14a protrude from the surface of the flow cell 14. One end serves as an inlet 14c for the supply of a sample solution, while the other end serves as an outlet 14d of the sample solution. Ligand molecules 16 are preliminarily bound onto the bottom of the channel 14a (see FIG. 3).

The flow cell 14 is replaceable to the sensor chip 12 in the measuring member 10, namely, the flow cell 14 can be disposable. The sensor chip 12 may be surface-modified, for example, with a silane coupling agent to facilitate replacement of the flow cell 14.

As described above, after the measuring member 10 is placed, the upper housing 81 is pivotally moved downwardly to close the unit, and thereby the detection window 86 faces the flow cell 14 and the optical fiber 40 lies over the closed fluid passage 14b in the flow cell 14, as illustrated in FIG. 3. One end of the optical fiber 40 is connected to the white-light source 20. The white-light source 20 is, for example, a halogen light source. The other end of the optical fiber 40 faces the detection window 86. The optical fiber 41 is connected to the spectrometer 30 at one end, and faces the detection window 86 at the other end. When the white-light source 20 is turned on, the white light enters the closed fluid passage 14b via the optical fiber 40 and the reflected light is detected by the spectrometer 30 via the optical fiber 41. The white-light source 20 and the spectrometer 30 are connected to the arithmetic and control unit 50, which controls the operations of these modules. The white-light source 20, the spectrometer 30, and the optical fibers 40 and 41 etc. configure a detection unit for detecting the intensity of the light reflected by the optical thin film where intermolecular interactions proceed. Note that the transmitted light may also be measured instead of the reflected light measured in the present embodiment. In the measurement of the transmitted light, the optical fiber for guiding the light from the light source and the optical fiber connected to the spectrometer are opposite to each other across the measuring member. The arithmetic and control unit 50 receives data indicating the spectral characteristics of the reflected light through an interface at a predetermined timing associated with the control of the detection operation in response to the execution of a program stored in a storage device described later. The arithmetic and control unit 50 also serves as an arithmetic unit for calculating a variety of values, described later, on the basis of the received data.

FIG. 4 schematically illustrates a circuit block diagram of the measuring system 1. With reference to FIG. 4, the arithmetic and control unit 50 includes a CPU 500, ROM 501, RAM 502, a storage device 503 such as a hard disk drive, a communication device 504, a reader/writer 505 for a storage medium such as a memory card, and an interface 506 for exchange of signals between the arithmetic and control unit 50 and the units of the measuring unit 80, the display, or the input device. A program for measuring intermolecular interactions is stored in the storage device 503. This program controls the CPU 500 to execute a variety of operations.

The operation of the measuring system 1 and a measuring method will now be described.

A sample solution 60 containing an analyte 62 is run from the inlet 14c through the closed fluid passage 14b to the outlet 14d, as illustrated in FIG. 3. At this time, the arithmetic and control unit 50 controls a solution feeder 35 (see FIG. 4) to feed the sample solution 60. The analyte 62 is a material which is to be specifically bound to a ligand 16, and is a target molecule to be detected. Examples of the usable analyte 62 include biomolecules such as proteins, nucleic acids, lipids and sugars, and foreign substances that are bound to biomolecules, such as drug substances and endocrine-disrupting chemicals.

The arithmetic and control unit 50 turns on the white-light source 20 before the flow of the sample solution 60 into the measuring member 10 and receives, from the spectrometer 30, spectral characteristics data that includes data indicating the intensity of the reflected light from the optical thin film (the SiN film 12b) before the start of intermolecular interactions.

The arithmetic and control unit 50 maintains the lighting of the white-light source 20 also during the flow of the sample solution 60 through the closed fluid passage 14b. White light travels through the flow cell 14 and reaches the sensor chip 12, and the reflected light is detected by the spectrometer 30. Information on the detected intensity of the reflected light detected by the spectrometer 30 is transmitted to the arithmetic and control unit 50.

As shown in FIGS. 5A and 5B, binding of the analyte molecules 62 in the sample solution 60 to the ligand molecules 16 changes the optical thickness 70 and thus the characteristics of the reflected light (e. g. , wavelength at the lowest intensity detected by the spectrometer 30). The arithmetic and control unit 50 receives, from the spectrometer 30, spectral characteristics data that includes data indicating the intensity of the reflected light during or after the progress of the intermolecular interactions.

The arithmetic and control unit 50, which receives the data on the intensity of the reflected light after the start of the intermolecular interactions, calculates a difference between the intensity of the reflected light before the start of the intermolecular interactions and the intensity during or after the progress. The arithmetic and control unit 50 then outputs the calculated value as an index indicating the degree of progress in the intermolecular interactions, and the value is displayed on the display 91 or stored in a storage medium with the reader/writer 505.

The intensity of the reflected light for use in the arithmetic is, for example, the spectral intensity of the reflected light, and a variation in spectral intensity of the reflected light between before and after the start of intermolecular interactions at a predetermined wavelength is defined as $\Delta I$. As illustrated in FIG. 6, when the distribution of the spectral intensity I of the reflected light shifts from the dashed line 161 before the start of intermolecular interactions to the solid line 162 after the progress of the intermolecular interactions, $\Delta I$ at the predetermined wavelength is calculated as an index indicating the degree of progress. The $\Delta I$ may be calculated at any wavelength at which the spectral intensity varies depending on the degree of progress in intermolecular interactions; however, a wavelength at which a variation in the spectral intensity increases depending on the degree of progress in intermolecular interactions, in particular, a wavelength associated with the largest difference facilitates the calculation of $\Delta I$.

The intensity of the reflected light used in the arithmetic is the spectral reflectance of the reflected light, for example, and the variation in spectral reflectance of the reflected light between before and after the start of intermolecular interactions at a predetermined wavelength is defined as $\Delta R$. As illustrated in FIG. 7, the distribution of the spectral reflectance R of the reflected light shifts from the dashed line 171 before the start of intermolecular interactions to the solid line 172 after the progress of intermolecular interactions, and $\Delta R$ at the predetermined wavelength is calculated as an index indicating the degree of progress. The $\Delta R$ may be calculated at any wavelength at which the spectral intensity varies depending on the degree of progress in intermolecular interactions; however, a wavelength at which a variation in the spectral intensity increases depending on the degree of progress in intermolecular interactions, in particular, a wavelength associated with the greatest variation in the spectral reflectance facilitates the calculation of $\Delta R$.

The principle of measuring the degree of progress in intermolecular interactions in the present embodiment will now be described. With reference to FIG. 12A, a model configuration is described which consists of a substance I (corresponding to the analyte 62), a substance II (corresponding to the ligand 16), an optical thin film III (corresponding to the SiN film 12b), and a substrate IV (corresponding to the silicon substrate 12a).

An interference film is composed of the substance I, the substance II, and the optical thin film III. The non-uniform layer consisting of the substance I and the substance II is now regarded as an effective uniform layer as shown in FIG. 12B. The thickness of the virtual uniform layer corresponds to the optical thickness to which the arithmetic is applied.

While the intermolecular interactions are proceeding from a state A in FIG. 13A to a state B in FIG. 13B, the reflectance curve $R(\lambda, \alpha)$ measured in any ongoing state can be expressed by Expression (1):

$$R(\lambda, \alpha) = (1-\alpha)A(\lambda) + \alpha B(\lambda) \quad . \quad . \quad . \quad \text{Expression (1)}$$

where $A(\lambda)$ is the reflectance curve in the state A, $B(\lambda)$ is the reflectance curve in the state B, and $\alpha$ is the degree of progress in the intermolecular interactions.

For example, at a degree of progress $\alpha$ of 60%, the value $R(\lambda 1, 0.6)$ of the reflectance at a wavelength $\lambda 1$ can be calculated from Expression (1): $0.4 \times A(\lambda 1) + 0.6 B(\lambda 1)$.

FIG. 14 shows a graph of reflectance curves $R(\lambda, \alpha)$ at degree of progress $\alpha$ of 0, 20, 40, 60, 80 and 100(%), i.e., a graph 201 ($\alpha$=0%), a graph 202 ($\alpha$=20%), a graph 203 ($\alpha$=40%), a graph 204 ($\alpha$=60%), a graph 205 ($\alpha$=80%) and a graph 206 ($\alpha$=100%).

In the graph of the spectral reflectance, the linearity of the variation $\Delta \lambda$ in the bottom wavelengths versus the degree of progress $\alpha$ is not necessarily ensured in this case; however, the linearity of the difference $\Delta R$ in the reflectance at a predetermined wavelength (e.g., $\lambda 1$) versus the degree of progress $\alpha$ is ensured as is apparent from the results of a simulation described later. It is noted that $\Delta R$ may be measured at any wavelength at which reflectance varies depending on the degree of progress.

Since the spectral reflectance R and the spectral intensity I have common variables, the same theory holds true even if the spectral reflectance R is replaced with the spectral intensity I.

In this manner, $\Delta I$ or $\Delta R$ is used as an index indicating the degree of progress in intermolecular interactions. $\Delta R$ is a value obtained by dividing the spectral intensity of a white-light source by the spectral intensity of the reflected light, and the spectral intensity of the white-light source is constant based on the device. Thus, the variation characteristics of $\Delta I$ and $\Delta R$ are the same, and either of them can be used as an index indicating the degree of progress in intermolecular interactions. Alternatively, a value containing the variable $\Delta I$, for example, $\Delta T = 1-\Delta R$ may be used as an index indicating the degree of progress in intermolecular interactions.

$\Delta I$, $\Delta R$, and $\Delta T$ each linearly vary with the degree of progress $\alpha$ in intermolecular interactions, as seen from FIG. 8 showing $\Delta I$, for example. These can be each used as an exact index indicating the degree of progress in intermolecular interactions.

Furthermore, each of ∆I, ∆R, and ∆T can be calculated without obtaining an approximate curve used for determining the bottom position, and thus the values can be easily calculated. Since these values reflect general shifts of the spectral characteristics of the reflected light, variations can be accurately detected.

∆I, ∆R, or ∆T may be calculated at only one predetermined wavelength or two or more different wavelengths as seen from FIGS. 6 and 7 showing ∆I and ∆R, respectively. As illustrated in FIG. 9, for example, the distribution of the spectral intensity I of the reflected light before the start of intermolecular interactions shifts from the dashed line 181 to the solid line 182 in accordance with the progress of intermolecular interactions. ∆I at two or more different wavelengths within a predetermined range is calculated at this time. In measurement of ∆I at a plurality of wavelengths, the highest-accuracy ∆I may be adopted as a measured value, or the measured value may be determined based on a plurality of ∆I values.

Alternatively, the sum of ∆Is, ∆Rs, or ∆Ts measured at a plurality of wavelengths within a predetermined wavelength range or the sum of the absolute values of ∆Is, ∆Rs, or ∆Ts may be calculated. For example, as illustrated in FIG. 10, the distribution of the spectral intensity I of the reflected light before the start of intermolecular interactions shifts from the dashed line 191 to the solid line 192 in accordance with the progress of intermolecular interactions. The sum of the absolute values of ∆Is within a predetermined wavelength range is calculated at this time.

Such a larger number of values acquired from multiple wavelengths can lead to higher accuracy of measurement. Furthermore, a general shift of the curve of spectral characteristics can be correctly detected, which also results in noise canceling. Consequently, a variation in intermolecular interactions can be advantageously measured with higher accuracy.

In the calculation of values at a single predetermined wavelength, applicability of a monochromatic-light source eliminates the need for an expensive spectrometer that detects spectral intensity. Furthermore, availability of a simply configured monochromatic-light source can lower the costs of the measuring system.

FIG. 11 illustrates an exemplary flow chart of a measuring program for causing the arithmetic and control unit 50, which is a computer, to execute a process.

The arithmetic and control unit 50 turns on the white-light source 20 to start detection by the spectrometer 30 and acquires, from the spectrometer 30, data on the spectral characteristics of the reflected light before the start of intermolecular interactions (step S1).

The arithmetic and control unit 50 then determines wavelength for the measurement of the difference in the spectral intensity of the reflected light (measurement wavelength) on the basis of the acquired data on the spectral characteristics of the reflected light (step S2). For example, wavelength corresponding to a range with a steep incline on a spectrum curve, such as wavelength between two adjacent extreme values, is selected as the measurement wavelength such that the progree of intermolecular interactions causes a great variation in the spectral intensity. If measurement wavelength is determined in advance, step S2 is unnecessary. Alternatively, after acquisition of data on the spectral characteristics after the start of intermolecular interactions, the measurement wavelength may be determined on the basis of spectral characteristics before and after the start of the intermolecular interactions.

The arithmetic and control unit 50 then controls the solution feeder 35 for the sample solution 60 to provide the flow of the sample solution 60 in the closed fluid passage 14b and acquires, from the spectrometer 30, data on the intensity of the reflected light during and/or after the progress of intermolecular interactions (step S3).

The arithmetic and control unit 50, which acquires the data on the intensity of the reflected light after the start of the intermolecular interactions, calculates a difference between the intensity of the reflected light before the start of the intermolecular interactions and the acquired intensity of the reflected light during or after the progress (step S4). The arithmetic and control unit 50 then outputs the calculated value as an index indicating the degree of progress in the intermolecular interactions, and the value is displayed on the display 91 or stored in the storage medium (step S5).

In place of or independently of step S4, the arithmetic and control unit 50 may calculate the sum of differences within a predetermined wavelength range or the sum of absolute values of the differences to output the resultant value.

Note that the program can be updated as appropriate via the communication device 504 connected through, for example, a LAN to public lines such as the Internet.

Following is the results of the simulation intended to support that the measurement of a difference in intensity of the reflected light or transmitted light between before and after the start of intermolecular interactions at a predetermined wavelength results in the generation of an exact index indicating the degree of progress in the intermolecular interactions. FIG. 15 illustrates the results of the simulation of the spectral reflectance of a model optical thin film. In this simulation, a SiN thin film having a thickness of 66.5 nm was provided on a silicon substrate having a thickness of 725 μm, and substances having a refractive index of 1.5 were deposited on the SiN thin film to a thickness of 650 nm. Pure water covered the substance deposited on the SiN thin film, and a pure water layer was assumed to have a thickness of 100 μm at the time of the degree of progress in the deposition reaching 100%. The measurement wavelength ranged from 500 to 700 nm. The curves depicted in FIG. 15 indicate the spectral reflectance from 0% (not attached) to 100% (650 nm attached) in progress with an increment of 10%.

The graphs in FIGS. 16 and 17 are derived from the data in FIG. 15. FIG. 16 illustrates the relationship between the

**EP 2 653 853 A1**

degree of progress and the difference Δλ in the bottom wavelength. Δλ denotes a difference from the bottom wavelength at the 0% degree of progress. FIG. 17 illustrates the relationship between the degree of progress and the difference ΔR in the reflectance. ΔR denotes a difference in the reflectance at a wavelength of 600 nm. ΔR also denotes a difference from the reflectance at the 0% degree of progress.

With reference to FIG. 16, the difference Δλ in the bottom wavelength varies nonlinearly with the degree of progress. In contrast, the difference ΔR in the reflectance varies linearly with the degree of progress, as illustrated in FIG. 17. Thus, the difference ΔR in the reflectance accurately indicates the degree of progress in intermolecular interactions. In addition, other simulations with different materials of the optical thin film or various refractive indices and thicknesses of the substances to be deposited gave the same results as those described above, which confirmed ensured linearity of the difference at a predetermined wavelength.

INDUSTRIAL APPLICABILITY

The present invention can be applied to measurement of the degree of progress in intermolecular interactions of biomolecules and organic polymers, etc.

EXPLANATION OF REFERENCE NUMERALS

| 1 | measuring system |
| 10 | measuring member |
| 12 | sensor chip |
| 12a | silicon substrate |
| 12b | SiN film |
| 14 | flow cell |
| 14a | channel |
| 14b | closed fluid passage |
| 14c | inlet |
| 14d | outlet |
| 16 | ligand |
| 20 | white-light source |
| 30 | spectrometer |
| 40, 41 | optical fiber |
| 50 | arithmetic and control unit |
| 60 | sample solution |
| 62 | analyte |
| 80 | measuring unit |
| I | spectral intensity |
| R | spectral reflectance |
| α | degree of progress in intermolecular interaction |
| λ | wavelength of reflected light |

**Claims**

1.   A measuring method of an intermolecular interaction for optically measuring an intermolecular interaction between a plurality of substances on a thin film, the method comprising:

   detecting intensity at a first wavelength of reflected light or transmitted light before start of the intermolecular interaction with a detection unit which detects the reflected light from the thin film or the transmitted light transmitted through the thin film;
   detecting intensity at the first wavelength of the reflected light or the transmitted light after the start of the intermolecular interaction with the detection unit; and
   calculating a difference in intensity of the reflected light or the transmitted light at the first wavelength between before and after the start of the intermolecular interaction.

2.   The measuring method of an intermolecular interaction of claim 1, wherein

9

the intensity of the reflected light is spectral intensity of the reflected light or a value containing the spectral intensity of the reflected light as a variable, or the intensity of the transmitted light is spectral intensity of the transmitted light or a value containing the spectral intensity of the transmitted light as a variable.

3. The measuring method of an intermolecular interaction of claim 1 or 2, further comprising calculating a difference in intensity of the reflected light or the transmitted light at a second wavelength which is different from the first wavelength.

4. The measuring method of an intermolecular interaction of claim 1 or 2, further comprising measuring a plurality of differences in intensity of the reflected light or the transmitted light at different wavelengths within a predetermined wavelength range and calculating a sum of the differences or a sum of absolute values of the differences.

5. A measuring system of an intermolecular interaction for optically measuring an intermolecular interaction between a plurality of substances on a thin film, the system comprising:

   a detection unit which detects reflected light from the thin film or transmitted light transmitted through the thin film, the detection unit detecting intensity at a first wavelength of the reflected light or the transmitted light before start of the intermolecular interaction, and detecting intensity at the first wavelength of the reflected light or the transmitted light after the start of the intermolecular interaction; and
   an arithmetic unit which calculates a difference in intensity of the reflected light or the transmitted light at the first wavelength between before and after the start of the  intermolecular interaction.

6. The measuring system of an intermolecular interaction of claim 5, wherein
the intensity of the reflected light is spectral intensity of the reflected light or a value containing the spectral intensity of the reflected light as a variable, or the intensity of the transmitted light is spectral intensity of the transmitted light or a value containing the spectral intensity of the transmitted light as a variable.

7. The measuring system of an intermolecular interaction of claim 5 or 6, wherein the arithmetic unit calculates a difference in intensity of the reflected light or the transmitted light at a second wavelength which is different from the first wavelength.

8. The measuring system of an intermolecular interaction of claim 5 or 6, wherein the arithmetic unit measures a plurality of differences in intensity of the reflected light or the transmitted light at different wavelengths within a predetermined wavelength range to calculate a sum of the differences or a sum of absolute values of the differences.

9. A program for causing a computer to execute a process for optically measuring an intermolecular interaction between substances on a thin film, the program causing the computer to execute:

   a process of detecting intensity at a first wavelength of reflected light or transmitted light before start of the intermolecular interaction with a detection unit for detecting the reflected light from the thin film or the transmitted light transmitted through the thin film;
   a process of detecting intensity at the first wavelength of the reflected light or the transmitted light after the start of the intermolecular interaction with the detection unit; and
   a process of calculating a difference in intensity of the reflected light or the transmitted light at the first wavelength between before and after the start of the intermolecular interaction.

10. The program of claim 9, wherein
the intensity of the reflected light is spectral intensity of the reflected light or a value containing the spectral intensity of the reflected light as a variable, or the intensity of the transmitted light is spectral intensity of the transmitted light or a value containing the spectral intensity of the transmitted light as a variable.

11. The program of claim 9 or 10, further causing the computer to execute a process of calculating a difference in intensity of the reflected light or the transmitted light at a second  wavelength which is different from the first wavelength.

12. The program of claim 9 or 10, further causing the computer to execute a process of measuring a plurality of differences in intensity of the reflected light or the transmitted light at different wavelengths within a predetermined wavelength range to calculate a sum of the differences or a sum of absolute values of the differences.

# FIG.1

# FIG.2

# FIG3

# FIG.4

EP 2 653 853 A1

# FIG.5A

BEFORE BINDING

# FIG.5B

AFTER BINDING

## FIG.6

SPECTRAL INTENSITY I

*161*

*162*

ΔI

WAVELENGTH λ

## FIG.7

REFLECTANCE R

*171*

*172*

ΔR

WAVELENGTH λ

## FIG.8

ΔI

DEGREE OF PROGRESS α

## FIG.9

SPECTRAL INTENSITY I

181

182

ΔI

ΔI

ΔI

WAVELENGTH λ

FIG.10

# FIG.11

```
         ┌──────────────┐
         │    START     │
         └──────────────┘
                 │
                 ▼
   ┌──────────────────────────────┐
   │      DETECT SPECTRAL         │────  S1
   │  CHARACTERISTICS BEFORE START│
   └──────────────────────────────┘
                 │
                 ▼
   ┌──────────────────────────────┐
   │    DETERMINE MEASUREMENT     │────  S2
   │         WAVELENGTH           │
   └──────────────────────────────┘
                 │
                 ▼
   ┌──────────────────────────────┐
   │ DETECT SPECTRAL CHARACTERISTICS│──  S3
   │  DURING AND/OR AFTER PROGRESS │
   └──────────────────────────────┘
                 │
                 ▼
   ┌──────────────────────────────┐
   │   CALCULATE DIFFERENCE AT    │────  S4
   │   MEASUREMENT WAVELENGTH     │
   └──────────────────────────────┘
                 │
                 ▼
   ┌──────────────────────────────┐
   │      OUTPUT DIFFERENCE       │────  S5
   └──────────────────────────────┘
                 │
                 ▼
         ┌──────────────┐
         │     END      │
         └──────────────┘
```

## *FIG.12A*

I

II

III

IV

## *FIG.12B*

I + II

III

IV

## FIG.13A

STATE A
DEGREE OF PROGRESS IN INTERACTION 0%

## FIG.13B

STATE B
DEGREE OF PROGRESS IN INTERACTION 100%

## FIG.14

EP 2 653 853 A1

# FIG.15

# FIG.16

EP 2 653 853 A1

EP 2 653 853 A1

## FIG.17

*FIG.18A*

104
112
102

*FIG.18B*

120
112
104
112
102

*FIG.18C*

130
120
112
104
102

# FIG.19

# FIG.20

# *FIG.21*

# FIG.22A

# FIG.22B

# *FIG.23*

<table>
<tr><td colspan="2" align="center"><strong>INTERNATIONAL SEARCH REPORT</strong></td><td>International application No.</td></tr>
<tr><td colspan="2"></td><td>PCT/JP2011/078123</td></tr>
</table>

A.  CLASSIFICATION OF SUBJECT MATTER
*G01N21/27*(2006.01)i, *G01N33/543*(2006.01)i

According to International Patent Classification (IPC) or to both national classification and IPC

B.  FIELDS SEARCHED

Minimum documentation searched (classification system followed by classification symbols)
G01N21/00-21/01, G01N21/17-21/74, G01N33/48-33/98

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X<br>Y | JP 2004-132799 A  (Hitachi, Ltd.),<br>30 April 2004 (30.04.2004),<br>entire text; all drawings<br>& US 2004/0070764 A1 | 1-3,5-7,9-11<br>4,8,12 |
| Y | JP 2008-64685 A  (Dainippon Printing Co., Ltd.),<br>21 March 2008 (21.03.2008),<br>paragraph [0039]<br>(Family: none) | 4,8,12 |
| Y | JP 2005-147896 A  (Kagawa University, Aoi Corp.,<br>AOI Electronics Co., Ltd.),<br>09 June 2005 (09.06.2005),<br>paragraph [0014]; fig. 4<br>(Family: none) | 4,8,12 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>29 February, 2012 (29.02.12) | Date of mailing of the international search report<br>13 March, 2012 (13.03.12) |
|---|---|
| Name and mailing address of the ISA/<br>Japanese Patent Office | Authorized officer |
| Facsimile No. | Telephone No. |

Form PCT/ISA/210 (second sheet) (July 2009)

| | INTERNATIONAL SEARCH REPORT | International application No. |
|---|---|---|
| | | PCT/JP2011/078123 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2007-292726 A  (Dainippon Screen Mfg. Co., Ltd.), 08 November 2007 (08.11.2007), paragraph [0091]; fig. 6 (Family: none) | 4,8,12 |
| A | JP 2002-365210 A  (Hitachi, Ltd.), 18 December 2002 (18.12.2002), entire text; all drawings (Family: none) | 1-12 |
| A | JP 2006-329631 A  (Hitachi, Ltd.), 07 December 2006 (07.12.2006), entire text; all drawings & US 2006/0263243 A1    & EP 1726939 A2 | 1-12 |

Form PCT/ISA/210 (continuation of second sheet) (July 2009)

33

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3786073 B

**Non-patent literature cited in the description**

- **SANDSTROM et al.** *APPL. OPT.,* 1985, vol. 24, 472